Europäisches Patentamt

European Patent Office

Office européen des brevets

(11) Publication number: **0 219 979 B1**

## EUROPEAN PATENT SPECIFICATION

(45) Date of publication of patent specification: **08.04.92**

(21) Application number: **86307244.3**

(22) Date of filing: **19.09.86**

Divisional application 90201942.1 filed on 19/09/86.

(51) Int. Cl.5: **A61K 37/02**, A61K 37/43, A61K 37/24

(54) Treating animals using IL-2, formulations therefor and their preparation.

(30) Priority: **20.09.85 US 778370**
**20.09.85 US 778371**
**20.09.85 US 778372**
**25.04.86 US 856035**
**25.04.86 US 856680**

(43) Date of publication of application:
**29.04.87 Bulletin 87/18**

(45) Publication of the grant of the patent:
**08.04.92 Bulletin 92/15**

(84) Designated Contracting States:
**AT BE CH DE FR GB IT LI LU NL SE**

(56) References cited:
**WO-A-86/05208**
**US-A- 4 518 584**

(73) Proprietor: **CETUS CORPORATION**
**1400 Fifty-Third Street**
**Emeryville California 94608(US)**

(72) Inventor: **Nunberg, Jack Henry**
**5933 Chabot Road**
**Oakland, CA 94618(US)**
Inventor: **Newell, Arthur Debney**
**45 Robert Road**
**Orinda, CA 94563(US)**
Inventor: **Doyle, Michael Varian**
**2709 Mountaingate**
**Oakland, CA 94611(US)**
Inventor: **White, Thomas James**
**5729 Clover Drive**
**Oakland, CA 94618(US)**

(74) Representative: **Bizley, Richard Edward et al**
**HEPWORTH LAWRENCE BRYER & BIZLEY**
**2nd Floor Gate House South West Gate**
**Harlow, Essex CM20 1JN(GB)**

EP 0 219 979 B1

**Description**

The invention is concerned with the use of interleuking-2 (IL-2) in the manufacture of a medicament enhancing the immune response of animals including humans to vaccines.

The use of vaccines to prevent diseases in farm livestock, sports animals and household pets is a common practice, and considerable effort has been, and is being, made to extend this practice to cover a more extensive array of diseases to which these animals are subject. For example, the use of rabies vaccine in animals is by now commonplace, and efforts are being made to obtain suitable vaccines to immunize animals against other diseases.

One problem that frequently is encountered in the course of active immunization is that the antigens used in the vaccine are not sufficiently immunogenic to raise the antibody titer to sufficient levels to provide protection against subsequent challenge or to maintain the potential for mounting these levels over extended time periods. Another problem is that the vaccine may be deficient in inducing cell-mediated immunity which is a primary immune defense against bacterial and viral infection. Notorious among such "weak" animal vaccines are those constituted from inactivated Haemophilus Pleuropneumoniae (Hpp) (which is associated with respiratory disease in pigs).

In order to obtain a stronger humoral and/or cellular response, it is common to administer the vaccine in a formulation containing an adjuvant (immunopotentiator), a material which enhances the immune response of the patient to the vaccine. The most commonly used adjuvants for vaccines are oil preparations and alum. The mechanisms by which such adjuvants function are not understood, and whether or not a particular adjuvant preparation will be sufficiently effective in a given instance is not predictable.

There is considerable background information available with respect to the biological activity of hIL-2. hIL-2 can be obtained from the supernatant of concanavalin-A (ConA) stimulated spleen cells or, presently, using recombinant technology, and has several measurable activities in vitro. First, it is a T-cell growth factor as measured by, for example, thymidine uptake when hIL-2 is added to cultures of cytotoxic or helper T-cell lines. It is mitogenic with respect to adult thymocytes, and stimulates a cytotoxic cell response (e.g., lymphokine-activated-killer (LAK) cell). It has also been shown to replace helper T-cells in athymic murine spleen cell cultures (Watson. J., et al, Immunological Rev (1980) 51:257-278). Specifically, in the presence of IL-2 and antigen, specific T helper cells are generated which are able to contribute to antibody responses. Presumably this occurs because hIL-2 is involved in the antigen-dependent maturation of helper T-cells in these nude mouse spleen cultures.

IL-2 has also been shown to directly affect B cells in vitro. Both proliferation and IgM and IgG secretion are enhanced by IL-2 in populations of purified, activated B cells (Mingari, M.C., et al., Nature (1984) 312:641; Mittler, R., et al., J. Immunol. (1985) 134: 2393-2399; Muraguchi, A., et al., J. Exp. Med. (1985) 161:181-197).

How these in vitro activities translate into a specific in vivo mechanism for mounting an immune defense is not clear. However, with respect to such in vitro studies, cross-reactivity among species of various IL-2s has been studied. For example, Redelman, D., et al, J Immunol Meth (1983) 56:359-370) show that hIL-2 supports activated T lymphocytes derived from rabbit and mouse to approximately the same extent as they are supported by the endogenous forms of IL-2. Ruscetti, F.W., et al, Blood (1981) 57:379-393 were the first to demonstrate the ability of hIL-2 to behave as a growth factor, not only for human T-cells, but also peripheral blood lymphocytes or splenocytes from other primates, horse, guinea pig, cat, rat, and mouse. Carter. J., et al (Fed Proc (1985) 44:1290) disclose the ability of hIL-2 to enhance the development and maintenance of bovine cytotoxic lymphocytes in vitro.

Doyle, M.V., et al, J Bio Resp Mod (1985) 4: 96-109 reports in vitro lymphocyte proliferation studies that compared the activities of native hIL-2 and a recombinant form of hIL-2 on human and animal lymphocytes. The native IL-2 and recombinant IL-2 exhibited the same range of activity on animal cells.

Some in vivo data are also available. The activity of IL-2 in vivo has been shown to restore immunocompetence in nude mice in response to heterologous erythrocytes (Stötter, H., et al, Eur J Immunol (1980) 10: 719-722). There is information concerning cross-species reactivity, as well. Reed, S.G., et al, J Immunol (1984) 133:3333, disclosed the ability of hIL-2 to reconstitute spleen cell responses in mice infected with a parasitic protozoan, and Farrar, J.J., et al, Immunol Rev (1982) 63:158, showed that in vivo injection of hIL-2 stimulates the splenic T-cells in nude mice. US-A-4 518 584 discloses the reconstitution of normal immunofunction in aged humans or animals by IL-2.

In summary, it is known that hIL-2 behaves in some manner in vivo to mediate a successful immune response, including a response to a specific antigen, and in vitro studies have shown that cross-species reactivity of hIL-2 is very diverse (prior in vivo cross-species studies have involved only murine subjects for hIL-2).

However, because the nature of IL-2 activity in vivo is not completely understood and because the mechanism of involvement of IL-2 in the immune response is not understood, it is not, at this time, possible to predict its effect on particular diseases, other therapeutic or prophylactic regimens, or metabolism. Accordingly, there is no suggestion in the art that hIL-2 would successfully enhance the efficacy of vaccines.

The main aspect of the invention is the use of an IL-2 for the manufacture of a medicament for enhancing the immune response of animals including humans to a vaccine.

When used as a vaccine adjuvant, hIL-2 is effective on humans as well as other animals. This involves administering hIL-2 medicament to the human as part of the vaccination regime.

Figure 1 shows the amino acid sequence of hIL-2.

Figures 2A and 2B are dose-response curves showing the results of the lymphocyte proliferation tests described in section C.1 of the Examples, infra.

Figure 3 shows the effect of hIL-2 on blastogenesis of bovine and porcine T-lymphocytes.

A. Definitions

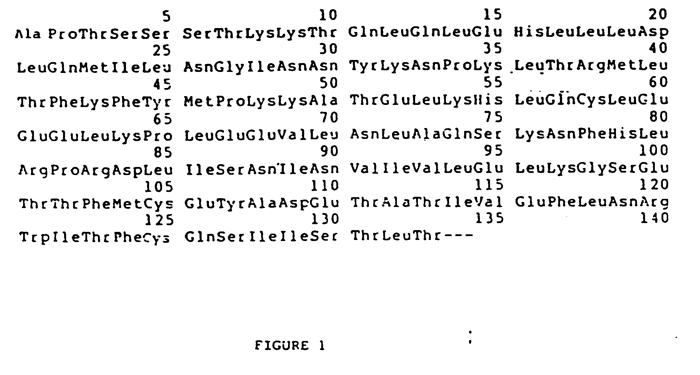

As used herein, "hIL-2" refers to a polypeptide exhibiting the spectrum of activities characterizing this protein. Specifically, the protein must be capable of stimulating the proliferation of hIL-2 dependent cytolytic and helper T cell lines, as set forth in the standard assays of Gillis, S, et al, J Immunol (1978) 120:2027-2032 and of Watson, J, J ExP Med (1979) 150: 1510-1519. The amino acid sequence of native hIL-2 is shown in Figure 1. This primary amino acid sequence may be obtained as the native protein from natural sources or may be recombinantly derived. other primary sequences of modest modification including deletion, addition, substitution or alterations of the amino acids of the sequence shown, which do not result in serious impairment of activity are, of course, included in the definition. For example, it is established that replacement of the cysteine at position 125 with a neutral amino acid results in a mutein of superior stability and satisfactory reactivity. (See U.S. Patent No. 4,518,584: Doyle, M.V., et al, supra.)

In addition, hIL-2, like any other protein, may exist in neutral or in salt form, and may contain associated nonprotein moieties in the nature of glycosylation, phosphorylation, or acetylation. These modifications, too, are included in the definition so long as biological activity is not destroyed thereby.

The word "IL-2" herein also includes bovine IL-2 as described by Cerretti et al., P.N.A.S. 83: 3223-3227 (1986)".

As used herein, the term "adjuvant" has its conventional meaning, i.e., the ability to enhance the immune response to a particular antigen. Such ability is manifested by a significant increase in immune-mediated protection. Enhancement of humoral immunity is typically manifested by a significant increase (usually > 10%) in the titer of antibody raised to the antigen.

B. General Method

The formulations of the invention are most conveniently administered by intramuscular injections or as sustained release compositions although other methods of administration are possible. Specific formulations to prevent hydrolysis during digestion would be necessitated for oral formulation, and intravenous injections are generally Uneconomic due to the skill level and care required in the administration. Therefore, formulations suitable for intramuscular injection, especially sustained release formulations, are preferred.

Standard formulations are either liquid injectables or solids which can be taken up in suitable liquids as suspensions or solutions for injection. Suitable excipients are, for example, water, saline, dextrose, glycerol, and ethanol. Nontoxic auxiliary substances, such as wetting agents, buffers, or emulsifiers may also be added. One specific useful formulation contains an effective amount of detergent, such as 0.1% sodium dodecyl sulfate (SDS), to effect solubility and bacteriostasis.

A variety of techniques are known in the art to effect long-term stability and slow release. For example, stability and half-life of hIL-2 are enhanced by coupling it to a hydrophilic polymer such as polyethylene glycol (PEG). This PEG-hIL-2 complex, called "PEGylated" hIL-2, is particularly useful for administering a single sustained action dose of hIL-2.

Sustained and continuous release formulations are of considerable variety, as is understood by those skilled in the art. An exemplary composition for sustained release parenteral administration is an injectable microcapsule formulation that with a single injection will deliver recombinant hIL-2 or soluble forms of hIL-2, such as PEGylated hIL-2, at a controlled rate of $10^3$ to $10^5$ units/kg/day (pure hIL-2 has a specific activity of 3-6 x $10^6$ u/mg). The microcapsule formulation is a free-flowing powder consisting of spherical particles 20 to 100 $\mu$m in diameter that can be injected intramuscularly or subcutaneously with a conventional

3

hypodermic needle, and the microcapsules consist of 0.5 to 5% hIL-2 encapsulated in poly(DL-lactide-co-glycolide) (DL-PLG) excipient, a biodegradable, biocompatible polyester. Alternative standard formulations for sustained release are also usable.

When used as a vaccine adjuvant, the hIL-2 will normally be administered separately from the vaccine, although it may, in some instances, especially in sustained or continuous release forms, be administered in combination with the vaccine. When hIL-2 is combined with the vaccine, the composition administered contains an immunogen that is effective in eliciting a specific response to a given pathogen or antigen, a pharmaceutically acceptable vaccine carrier and an immunopotentiating amount of hIL-2. A preferred regimen is to administer the hIL-2 continuously until 5 to 30 days, preferably 5 to 14 days, post-vaccination at levels above $10^3$ and below $10^6$ units/ kg/day. The term "continuously" is intended to denote true continuous administration, such as is achieved via a sustained release dosage form as well as a multiplicity of intermittent administrations of hIL-2 (or enhanced half-life forms of hIL-2 such as PEGylated hIL-2) that provide a pharmacokinetic pattern that mimics that achieved by true continuous administration. Data generated to date using daily intramuscular injections indicate a preferred dose is $10^4$ to $10^5$ units/kg/day. The vaccine will normally be administered per manufacturer's instructions. Other adjuvants may be administered either with the vaccine or together with the hIL-2.

The hIL-2 will typically be used to enhance the protection afforded by animal or human vaccines that are considered "weak" (i.e., provide diminished protection in terms of level, extent, and/or duration). Examples of such vaccines are bacterins such as Bordetella bacterin, Escherichia coli bacterins, Haemophilus bacterins, Leptospirosis vaccines, Moraxella bovis bacterin, Pasteurella bacterin and Vibrio fetus bacterin and attenuated live or killed virus products such as bovine respiratory disease vaccine (infectious bovine rhinotracheitis, parainfluenza, respiratory syncytial virus), bovine virus diarrhea vaccine, equine influenza vaccine, feline leukemia vaccine, feline respiratory disease vaccine (rhinotracheitis-calici-pneumonitis viruses), canine parvovirus vaccine, transmissible gastroenteritis vaccine, and pseudorabies vaccine.

C. Examples

The following examples are intended to further support or illustrate but not to limit the invention.

C.1. In Vitro Activity

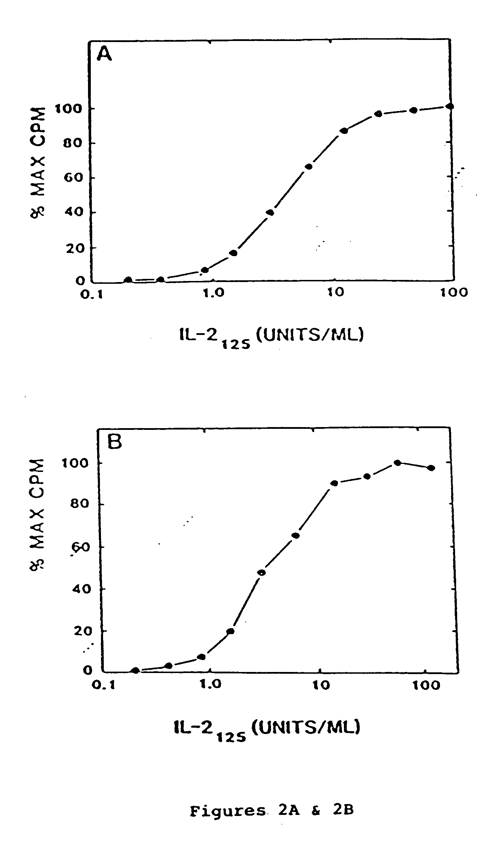

In vitro activity with respect to bovine and porcine peripheral blood mononuclear cells (PBMC) has been shown for recombinant hIL-2 (Fong, Susan, et al, Vet Immunol and Immunopathol (1986) 11:91-100). The hIL-2 used in this work is designated des-alanyl-rIL-2$_{ser125}$,lacks an initial alanine, and has a serine rather than a cysteine at position 125. It was shown to be mitogenic for unactivated bovine and porcine PBMC, and to be able to maintain the long-term growth of ConA-activated PBMC from both species. Figures 2A and 2B are curves showing the dose-response of ConA-activated bovine (2A) and porcine (2B) PBMC to des-alanyl-rIL$_{ser125}$.Also, bovine and porcine PBMC preincubated with des-alanyl-rIL-2$_{ser125}$ for 1-5 days showed enhanced cytotoxicity against tumor cell targets.

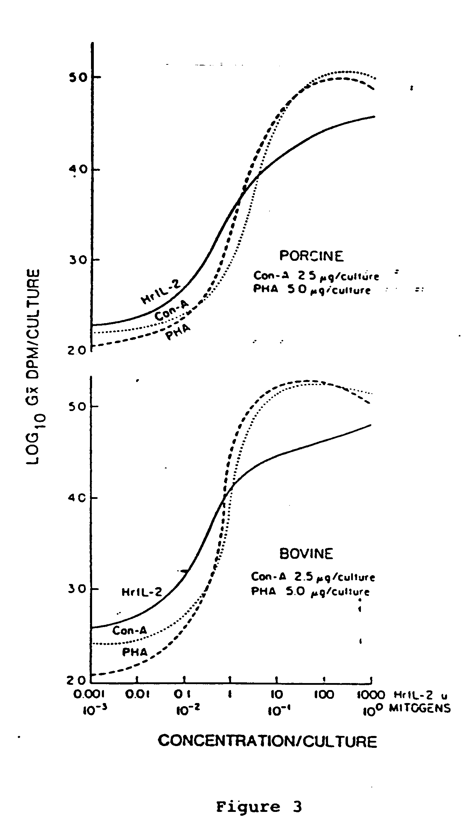

In addition, Stott, J.L., et al (in press) have shown that bovine and porcine peripheral blood lymphocytes were responsive to human recombinant IL-2 in lymphocyte blastogenesis assays. Blastogenesis was determined by incorporation of $^3$H-thymidine (18 hr pulse) in 4-day lymphocyte cultures, and the results expressed as the $\log_{10}$ of the geometric mean ($G_x$) of disintegrations per minute (DPM)/culture and plotted by nonlinear regression analysis as shown in Figure 3. Mitogen dilution and concentration of hIL-2 in units are shown on the X-axis. These results show that the effect of hIL-2 on bovine and porcine cells is comparable to that shown by the plant lectins PHA and ConA, which are known to stimulate blastogenesis.

C.2. Potentiation of Cell-Mediated Immunity

Since respiratory diseases are predominantly controlled by the cellular (T-cell) immune system, the ability of hIL-2 to boost the cellular immune response in livestock is indicative of its effectiveness against these symptomologies. In vivo injections of recombinant hIL-2 produced elevated levels of lymphocyte blastogenesis in the blood of calves.

Specifically, eight calves weighing 135-225 kg (3-5 months old) were randomly sorted into 4 groups of 2 each which received weekly injections for one month as follows: Groups 1, 2, and 3 received $10^4$, $10^5$, and $10^6$ units/kg, respectively, intramuscularly; group 4 received only excipient. The animals were assessed for lymphocyte stimulation. The results show that resting lymphocyte activity was elevated by the recombinant hIL-2 treatment as determined by blastogenesis assays performed prior to each inoculation over the period in calves receiving $10^5$ and $10^6$ units/kg only. For calves receiving $10^5$ units/kg, lymphocyte activity returned to normal within two weeks following the last IL-2 administration; $10^6$ units/kg-injected calves remained elevated at that time.

C.3. Effectiveness as an Adjuvant in Porcine Vaccine

Recombinant hIL-2 was shown to enhance the efficacy of an inactivated Hpp bacterin using 12 feeder pigs divided into 6 groups of 2 pigs each. Group 1 was an hIL-2 adjuvant control; group 2 was a bacterin control; group 3 received $10^3$ units hIL-2/kg as a single injection on days 0 and 21; group 4 received 5 daily injections of $10^3$ units/kg each following each vaccination; group 5 received 1 injection of $10^5$ units/kg on days 0 and 21; and group 6 received 5 daily injections of $10^5$ units/kg each following each vaccination.

The pigs in groups 2-6 were administered formalin-inactivated Hpp emulsified in an oil adjuvant intramuscularly in the neck muscles on days 0 and 21. The pigs in all groups were challenged on day 41 intranasally with serotype 1 Hpp and were killed on day 71 and autopsied. Lung area affected was determined visually with particular attention given to lung lesions. The pigs were weighed periodically during the 71 days --with weight gain being an indication of general state of health. The results were as follows:

| Group | Autopsy (% Lung Area Affected) | Average Rate of Body Weight Gain (kg/day) | |
|---|---|---|---|
| | | Days 0-41 | Days 41-71 |
| 1 | 24,57% | 0.69 | 0.30 |
| 2 | 6,100% | 0.69 | * |
| 3 | 25,58% | 0.62 | 0.27 |
| 4 | 12,12% | 0.55 | 0.86 |
| 5 | 13,19% | 0.62 | 0.39 |
| 6 | 0,0% | 0.58 | 0.88 |

*pig died 5 days after challenge

As shown by these results the groups that received daily post-vaccination injections of hIL-2 (groups 4 and 6) exhibited substantially higher weight gain post-challenge than did the groups treated otherwise. These results also show a significant reduction of lung pathology in groups 4 and 6, indicating increased protection against challenge provided by the daily administration of hIL-2 in the vaccination regimen. All animals showed high antibody titers against Hpp after challenge.

A second study was carried out to confirm the ability of hIL-2 (des-alanyl-rIL-2$_{ser125}$) to act as an adjuvant to Hpp vaccination. The protocol for the second study was similar to that of the first study described above: animals were vaccinated with/without hIL-2 treatment and subsequently challenged with virulent Hpp. Principal measures of efficacy included clinical signs following infection, weight gain following infection, and the extent of lung involvement at necropsy. The results of this second study are tabulated below.

| Group (N) | Ave. Percent Lung Affected At Necropsy | Ave. Daily Weight Gain (kg/day) | |
|---|---|---|---|
| | | Pre-Challenge | Post-Challenge |
| Control (2) | 34 | 0.37 | 0.31 |
| Hpp Alone (3) | 19 | 0.34 | 0.62 |
| Hpp + IL-2 (3) $10^5$ u/kg daily | 01 | 0.29 | 0.84 |
| Hpp + IL-2 (2) $10^4$ u/kg daily | 00 | 0.43 | 0.75 |
| No Challenge (2) | -- | 0.36 | 0.83 |

The data from the second study confirm the efficacy observed at $10^5$ u/kg/day hIL-2 in the first study and extend these findings to the lower dose of $10^4$ u/kg/day. Protection at an IL-2 dose of $10^4$ u/kg/day was comparable to that observed at the higher dose.

C.4. Effectiveness as an Adjuvant in Dogs

Dogs were injected with keyhole limpet hemocyanin (KLH) at the time of initial hIL-2 treatment and 7 days subsequently at the start of 5 days of (daily) hIL-2 treatment. Enzyme-linked immunoabsorbent assays (ELISAs) were performed on sera taken from the dogs to measure antibody response to KLH. A significant, dose-dependent increase in IgG antibody against KLH was observed in the IL-2 treated dogs. The increase was specific to the KLH immunogen used.

**Claims**

1. The use of an IL-2 for the manufacture of a medicament for enhancing the immune response of animals including humans to a vaccine.

2. The use claimed in Claim 1 wherein the IL-2 is hIL-.

3. The use claimed in Claim 2 wherein the hIL-2 is a water soluble form of hIL-2.

4. The use claimed in Claim 3 wherein the hIL-2 is PEGylated hIL-2.

5. The use claimed in any one of Claims 2, 3 or 4 wherein the IL-2 is des-alanyl-rIL-2$_{ser125}$.

6. The use claimed in any one of Claims 1 to 5 wherein the IL-2 is in the form of a continuous release formulation or is in the form of a single sustained action formulation.

7. The use claimed in Claim 6 wherein the IL-2 is to be in the form of a continuous release formulation and the IL-2 is to be administered continuously at a rate above $10^3$ and below $10^6$ units/kg/day.

**Revendications**

1. Utilisation d'une IL-2 pour la fabrication d'un médicament destiné à accroître la réponse immune d'animaux, y compris l'homme, à un vaccin.

2. Utilisation selon la revendication 1, dans laquelle l'IL-2 est une hIL-2.

3. Utilisation selon la revendication 2, dans laquelle la hIL-2 est une forme soluble dans l'eau de hIL-2.

4. Utilisation selon la revendication 3, dans laquelle la hIL-2 est une hIL-2 associée au polyéthylène glycol.

5. Utilisation selon l'une quelconque des revendications 2 à 4, dans laquelle la IL-2, est la des-alanyl-rIL-2$_{ser125}$.

6. Utilisation selon l'une quelconque des revendications 1 à 5, dans laquelle la IL-2 est sous la forme d'une composition à libération continue, ou sous la forme d'une simple composition à action prolongée.

7. Utilisation selon la revendication 6, dans laquelle la IL-2 est destinée à être sous la forme d'une composition à libération continue et la IL-2 est destinée à être administrée de manière continue à raison de plus de $10^3$ et de moins $10^6$ U/kg/jour.

**Patentansprüche**

1. Verwendung von IL-2 zur Herstelllung eines Arzneimittels zur Steigerung der Immunantwort von Tieren einschließlich Menschen auf einen Impfstoff.

2. Verwendung nach Anspruch 1, wobei das IL-2 hIL-2 ist.

3. Verwendung nach Anspruch 2, wobei das hIL-2 eine wasserlösliche Form von hIL-2 ist.

4. Verwendung nach Anspruch 3, wobei das hIL-2 PEG-yliertes hIL-2 ist.

**5.** Verwendung nach einem der Ansprüche 2, 3 oder 4, wobei das IL-2 des-alanyl-rIL-2$_{ser125}$ ist.

**6.** Verwendung nach einem der Ansprüche 1 bis 5, wobei das IL-2 in Form einer Zubereitung mit kontinuierlicher Freisetzung oder in Form einer einzelnen langzeitwirkenden Zubereitung vorliegt.

**7.** Verwendung nach Anspruch 6, wobei das IL-2 in Form einer Zubereitung mit kontinuierlicher Freisetzung vorliegen muß, und das IL-2 kontinuierlich in einer Dosierung von mehr als $10^3$ und weniger als $10^6$ Einheiten/kg/Tag verabreicht werden muß.

```
                5               10              15              20
 Ala ProThrSerSer SerThrLysLysThr GlnLeuGlnLeuGlu HisLeuLeuLeuAsp
                25              30              35              40
 LeuGlnMetIleLeu AsnGlyIleAsnAsn TyrLysAsnProLys LeuThrArgMetLeu
                45              50              55              60
 ThrPheLysPheTyr MetProLysLysAla ThrGluLeuLysHis LeuGlnCysLeuGlu
                65              70              75              80
 GluGluLeuLysPro LeuGluGluValLeu AsnLeuAlaGlnSer LysAsnPheHisLeu
                85              90              95              100
 ArgProArgAspLeu IleSerAsnIleAsn ValIleValLeuGlu LeuLysGlySerGlu
                105             110             115             120
 ThrThrPheMetCys GluTyrAlaAspGlu ThrAlaThrIleVal GluPheLeuAsnArg
                125             130             135             140
 TrpIleThrPheCys GlnSerIleIleSer ThrLeuThr---
```

FIGURE 1

Figures 2A & 2B

Figure 3